# EUROPEAN PATENT SPECIFICATION

(11) **EP 4 267 028 B1**
(45) Date of publication and mention of the grant of the patent: **12.02.2025**
(21) Application number: 21840799.7
(22) Date of filing: 16.12.2021
(51) Int. Cl.: A61B 18/24

(54) **SYSTEMS FOR VALVE RESECTION AND RESHAPING CATHETER/LASER-BASED VALVE LEAFLET REMOVAL**
SYSTEME ZUR KLAPPENRESEKTION UND UMFORMUNG EINER KATHETER-/LASERBASIERTEN KLAPPENSEGELENTFERNUNG
SYSTÈMES DE RÉSECTION DE VALVULE ET DE REMODELAGE DE RETRAIT DE FEUILLET DE VALVULE BASÉ SUR CATHÉTER/LASER

(30) Priority: 22.12.2020 US 202063129458 P
(43) Date of publication of application: 01.11.2023
(73) Proprietor: Koninklijke Philips N.V., 5656 AG Eindhoven (NL)
(72) Inventor: FRANCIS, Nathan C, 5656 AG Eindhoven (NL); ANDERSON, Michael, 5656 AG Eindhoven (NL); GRACE, Kenneth Peter, 5656 AG Eindhoven (NL)
(74) Representative: Philips Intellectual Property & Standards
(86) International application number: PCT/EP2021/086048
(87) International publication number: WO 2022/136079

(56) References cited:
- US-A1- 2015 342 629
- US-A1- 2019 321 103

## Description

### TECHNICAL FIELD

The technical field generally relates to endovascular procedures and more particularly relates to systems and methods for use in a Transvenous/transcatheter Aortic Valve Replacement (TAVR) procedure.

### BACKGROUND

Vascular catheters for laser cutting of vascular tissue are known from US2019/0321103 A1 and US2015/0342629 A1. Transcatheter aortic valve replacement (TAVR) is a minimally invasive heart procedure to replace a narrowed aortic valve that fails to open properly (i.e., aortic valve stenosis). The transcatheter aortic valve replacement is also called transcatheter aortic valve implantation (TAVI). The treatment by transvenous and transcatheter aortic valve repair (TAVR) is becoming more prevalent and accepted as a treatment option for patients because widespread training has made available the treatment option to medical personnel. Hence, more medical personnel have gained first-hand knowledge of this newer advanced medical procedure and recognize the patient benefits that can be realized.

The TAVR procedure allows for the implanting (i.e., replacing) of a heart valve without having to open the chest cavity. The resultant minimal invasive surgery for a heart valve replacement makes surgical valve replacement are more feasible treatment plan. This is because TAVR can now be considered an option for patients considered at intermediate or high risk of complications from traditional open-chest surgical aortic valve replacement.

However, there are differences in both approaches; for example, in the open chest replacement surgery, the degraded valve is completely removed. In TAVR, the damaged, native valve is left in place. The valve can have anatomical abnormalities, calcification, or infection. However, inserting a new valve over the native valve can cause complications in the TAVR procedure, including valve migration, valve embolization, paravalvular leakage, and blockage of the coronary arteries restricting blood flow to the heart. These complications can also occur, sometimes more frequently, during a valve-in-valve TAVR, where the transcatheter valve is deployed within a previously implanted bioprosthetic valve.

Therefore, deploying a TAVR valve on top of the existing damaged valve, either native or bioprosthetic, may not be performed because of the expected complications in the procedure. To alleviate the complications from implantation of a new valve, a catheter may be used to remove old valve leaflets at the location to prepare the implant site for a cleaner valve deployment and operation.

It is desired for a catheter configured to remove the old valve leaflets and prepare the site for the new valve replacement procedure.

Accordingly, technologically improved systems and methods for valve resection and reshaping using a catheter with an ablation tool to perform valve leaflet removal in a TAVR procedure is desirable. The following disclosure provides these technological enhancements, in addition to addressing related issues.

### BRIEF SUMMARY

The invention and its most advantageous embodiments are defined in the appended set of claims.

This summary is provided to describe select concepts in a simplified form that are further described in the Detailed Description. This summary is not intended to identify key or essential features of the claimed subject matter, nor is it intended to be used as an aid in determining the scope of the claimed subject matter.

In one exemplary embodiment, an apparatus including a leaflet resection catheter for a Transcatheter Aortic Valve Replacement (TAVR) procedure is provided. The apparatus includes a catheter configured at a distal end with a guidewire to deploy the catheter within a vessel lumen to a situs of an aortic valve for leaflet resection; an accessory tool configured with a set of grasping elements attached at a catheter's distal end to enable the accessory tool to travel down the vessel lumen to the situs of the aortic valve to exert a pulling action to draw the aortic valve leaflet in a direction towards the catheter's distal end to draw a portion of the aortic valve leaflet into a protective sleeve of the catheter at the catheter's distal end, and one or more fibers of a ring of fibers surrounding the catheter's distal end to resect tissue that includes a portion of the aortic valve leaflet drawn into the protective sleeve at the catheter's distal end.

Furthermore, other desirable features and characteristics of the system and method will become apparent from the subsequent detailed description and the appended claims, taken in conjunction with the accompanying drawings and the preceding background.

### BRIEF DESCRIPTION OF THE DRAWINGS

The present application will hereinafter be described in conjunction with the following drawing figures, wherein like numerals denote like elements, and:
FIG. 1 is an illustration of an exemplary diagram of combination tool of an apparatus for use in a TAVR procedure of the guidewire lumen, accessory tool, and pull-push actionlaser cutting catheter implemented by the TAVR system, in accordance with an exemplary embodiment;
FIG. 2 illustrates an exemplary diagram of another embodiment of a separate apparatus for use in a TAVR procedure of a combination of an accessory tool and cutting catheter implemented by the TAVR system, in accordance with an embodiment; and
FIG. 3 illustrates an exemplary flowchart for a method system of a leaflet resection catheter for use in a TAVR procedure, in accordance with an exemplary embodiment.

### DETAILED DESCRIPTION

The following detailed description is merely illustrative in nature and is not intended to limit the embodiments of the subject matter or the application and uses of such embodiments. As used herein, the word "exemplary" means "serving as an example, instance, or illustration." Thus, any embodiment described herein as "exemplary" is not necessarily to be construed as preferred or advantageous over other embodiments. The embodiments described herein are exemplary embodiments provided to enable persons skilled in the art to make or use the invention and not to limit the scope of the invention that is defined by the claims. Furthermore, there is no intention to be bound by any expressed or implied theory presented in the preceding technical field, background, summary, or the following detailed description.

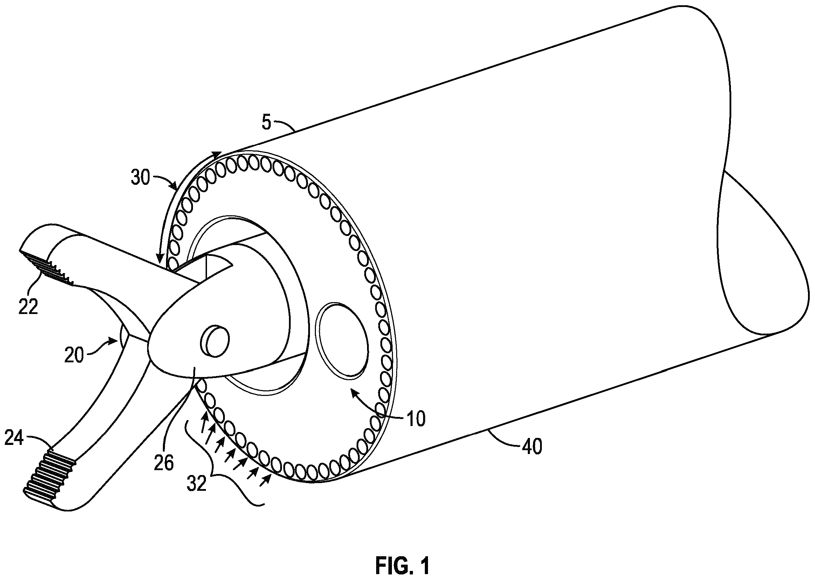

Exemplary embodiments provide a technical solution to this problem in the form of a leaflet resection catheter deployment system for use in a TAVR procedure (FIG. 1) embodying novel rules, vascular anatomy design factors, and recommended treatment protocols for deploying a combination apparatus including a catheter, accessory tool, and catheter cutting apparatus in a vessel lumen, as follows:

Provided embodiments include an improved catheter-based procedure for deploying a new TAVR valve on top of an existing damaged valve by the medical personnel gaining access to the heart valve, and inserting the catheter, and tracking it to the position. The catheter cutting apparatus is configured to excise valve leaflets and prepare the site for valve replacement. The outer sheath of the catheter is pulled back, exposing the grasping catheter to grab the leaflet tissue, and to draw the ring of fibers to ablate the leaflet from either the native or bioprosthetic valve.

Provided embodiments disclosed enable the performance of a medical procedure using an accessory tool for the forwardly grasping of tissue (i.e., the leaflets). The accessory tool is configured as a grasping catheter that can enable the user to pull a grasped leaflet into a catheter sheath and the excising of the leaflet tissue within the cutting sheath to prevent damage to surrounding tissue.

The figures and descriptions below provide more detail.

Turning now to FIG. 1, in an embodiment, the system for a leaflet resection catheter deployment by a catheter system 100 for use in a TAVR procedure in a vessel (also referred to herein as "system" 100) is depicted as associated with a vessel lumen (not shown). In various embodiments, the vessel lumen is a blood vessel in a patient. As mentioned, system 100 embodies bidirectional motion to perform tissue ablation of tissue with a catheter sheath at a situs in a vessel lumen. The catheter 5 may be manually operated by a user; manual input can include a direction and a placement operation.

The direction is generally, from the perspective of the distal tip of the catheter 5, forward and aft, longitudinally, within a vessel lumen. To perform TAVR, the doctor may access your heart through a blood vessel in your leg or through a tiny incision in your chest. The doctor may use other approaches to access your heart. A hollow tube (catheter 40) is inserted through the access point. Your medical provider can use various advanced imaging techniques to guide the catheter 40 through a vessel lumen to the heart valve location for rescission of the leaflet tissue at the valve location. The deployment system 100 includes a guidewire lumen 10, an accessory tool 20, and a opposite directionlaser cutting catheter 30. FIG. 1 illustrates an exemplary embodiment of the distal tip of the catheter system 100, featuring a guidewire lumen 10 to track the catheter 40 to the valve (not shown) in question, accessory tool lumen for the grasping catheter to travel to the valve in question, and in this embodiment, a ring of fibers for laser transmission to ablate the leaflet tissue from either the native or bioprosthetic valve.

The accessory tool 20 includes proximal elements 22, 24 (or grasping elements) and a distal element 26, which protrudes outward from the catheter 40. The proximal elements 22, 24 are coupled together on the distal side to enable the proximal elements to be positionable on opposite sides of the leaflets (not shown) to capture or retain the leaflets therebetween.

The mitral valve is composed of two leaflets, the anterior leaflet, which is a semi-circular shape and attaches to two-fifths of the annular circumference. There is continuity between the anterior leaflet of the mitral valve and the left and non-coronary cusp of the neighboring aortic valve, referred to as the aortic-mitral curtain. These two components of the aorto-mitral curtain are on two separate anatomical planes, situated at an angle of 120°, which corresponds to the planes of the aortic and mitral annulus, respectively, which can be grasped because of the angular location by the proximal elements 22, 24. When the flaps (i.e., leaflets) of the mitral valve do not close tightly enough, it causes blood to leak backward into the left atrium. This occurs due to valve leaflets bulging back - a condition called mitral valve prolapse and can be corrected by the valve replacement. The proximal elements 22, 24 may be made of cobalt-chromium, nitinol, or stainless steel, and the distal elements 26 can also be made of cobalt-chromium and stainless steel, or another material. In the alternative exemplary embodiment, the accessory tool can be a vacuum suction (instead of proximal elements) to grasp the aortic valve leaflet during the ablation of tissue of the aortic valve leaflet. The accessory tool is configured to pass through an introducer (not shown) of at least a range of 14f to 18f for use with a standard TAVR deployment catheter. In an exemplary embodiment, a sheath introducer is a long, wide bore, single lumen catheter with a wide plastic hub on the proximal end, which has a central smaller hole (one-way-valved to prevent back-flow of blood), through which various other vascular catheters can be inserted. In an exemplary embodiment, the sheath introducer comes in multiple diameters and lengths.

The laser cutting catheter 30 includes a ring of fibers 32 in a plurality of arrangements of an entirety, a semicircle, a one-third circle, or another fractional circle of the circumference of the catheter 40 distal end to ablate tissue of the aortic valve leaflet. The laser cutting catheter 30, in another exemplary embodiment, can reshape a valvular structure to better fit a replacement valve at the situs of the valve replacement. Also, a power source (not shown) is connected at a proximal end of the catheter 40 to energize a laser (via the fibers 32) to ablate tissue of the aortic valve leaflet grasped by the proximal elements 22, 24.

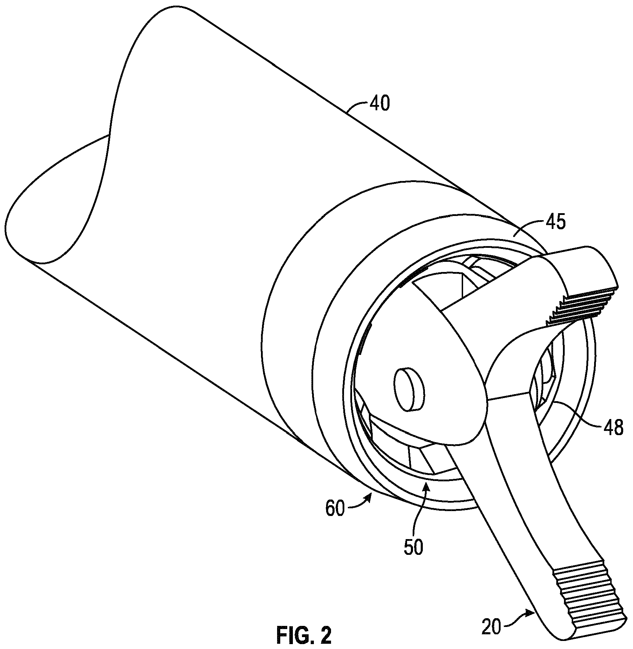

Turning to FIG. 2, an exemplary diagram of another embodiment of the stent deployment system is illustrated in accordance with an embodiment. In FIG. 2, The main components for the invention are a cutting sheath 50 to resect the valve leaflets, any type of power source (not shown) to energize the cutting mechanism on the cutting sheath 50, and a grasping catheter (or accessory tool) 20 to travel down the lumen (interior of cutting sheath 50) of the cutting sheath and grasp the leaflets. In FIG. 2, the cutting sheath 50 catheter 40 could also be a manual or powered mechanical cutter within a protective sleeve 45 to prevent unwanted tissue damage during deployment. The outer cutting sheath 48 will have a hemostatic valve (i.e., a valve to keep blood within the vessel lumen or to stop any bleeding) on the proximal end to allow tool pass-through while sealing off arterial pressures if an arterial approach is used. The catheter 40 will need to pass through a 14F-18F introducer, which is standard for a TAVR deployment catheter 40. In the exemplary embodiment, a vacuum suction instead of the grasping accessory tool 20 could be used to grasp, or retain the valve leaflet during cutting by the cutting sheath 50. In an exemplary embodiment, the cutting sheath 50 can also be implemented to reshape a valvular structure to better fit a replacement valve at the location in the vessel lumen. The catheter 40 can also be inserted via a transvenous approach or transapical approach.

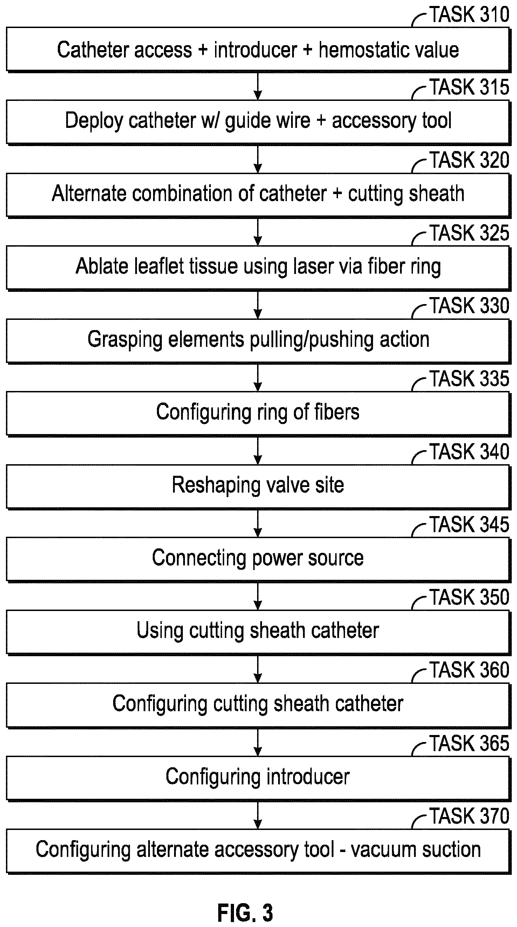

FIG. 3 illustrates an exemplary flowchart of the TAVR method for using the catheter configured with the accessory tool and cutting sheath in accordance with various embodiments. In an exemplary embodiment, FIG. 3 illustrates a method for deployment of a combination catheter, accessory tool, and cutting sheath structure or laser ablation tool in a vessel lumen. At task 310, the user (i.e., healthcare provider) gains access to an artery or vein via an introducer. Also, the catheter can be configured with a hemostatic valve on the proximal end to enable the accessory tool to pass through the vessel lumen whilst sealing off arterial pressures. At task 315, the user inserts the catheter that is configured at a distal end with a guidewire for deploying the catheter within a vessel lumen to a situs of an aortic valve for leaflet resection. The catheter at task 315 is also configured with an accessory tool of a set of grasping elements attached at the catheter's distal end to enable the accessory tool to travel down the vessel lumen to the situs of the aortic valve. The accessory tool enables the grasping of an aortic valve leaflet between the set of grasping elements of the accessory tool at the catheter's distal end to exert a pulling action to draw the aortic valve leaflet in a direction towards the catheter's distal end.

In an alternative exemplary embodiment, at task 320, the user inserts a catheter configured at a distal end with an accessory tool including a set of grasping elements attached at a catheter's distal end to enable the accessory tool to travel down a vessel lumen to a situs of the aortic valve. The accessory tool grasps an aortic valve leaflet between the set of grasping elements of the accessory tool at the catheter's distal end to exert a pulling action to draw the aortic valve leaflet in a direction towards the catheter's distal end. Further, the accessory tool by the pulling action draws a portion of the aortic valve leaflet into a protective sleeve of the catheter at the catheter's distal end. The cutting sheath catheter resects tissue of a portion of the aortic valve leaflet drawn into the protective sleeve at the catheter's distal end. The tissue resection is of tissue of a portion of the aortic valve leaflet that has been drawn within the protective sleeve and held between the set of grasping elements.

At task 325, one or more fibers transmit the laser to ablate tissue whilst the aortic valve leaflet is held by the set of grasping elements within the protective sleeve, thereby preventing tissue damage outside the protective sleeve. At task 330, in response pulling action of the accessory tool while grasping an aortic valve leaflet tissue between the set of grasping elements, enabling a reactive action of simultaneous pulling the leaflet in a direction into the protective sleeve whilst moving the distal end of the catheter in the opposite direction towards the aortic valve leaflet that is drawn into the protective sleeve at the catheter's distal end. At task 335, the ring of fibers about a circumference of the catheter's distal end in entirety or part of enable the ablating of tissue of the aortic valve leaflet. At task 340, laser transmitted via the fibers enables the reshaping of a valvular structure to better fit a replacement valve at the situs. At task 345, a power source is connected at a proximal end of the catheter to energize the laser for ablating the tissue of the aortic valve leaflet.

In an alternative exemplary embodiment, at task 350, the cutting sheath catheter is powered mechanical cutter located within the protective sleeve to prevent unwanted tissue damage.

At task 360, the cutting sheath catheter is configured as an outer cutting sheath with a hemostatic valve on a proximal end of the catheter to enable the accessory tool to pass through the vessel lumen whilst sealing off arterial pressures. At task 365, the accessory tool is configured to pass through an introducer of at least a range of 14f to 18f for use with a standard TAVR deployment catheter.

At task 370, in an alternate exemplary embodiment, the catheter is configured with an accessory tool that includes a vacuum suction to grasp the aortic valve leaflet during the ablation of tissue of the aortic valve leaflet.

Those of skill in the art will appreciate that the various illustrative logical blocks, modules, circuits, and algorithm steps described in connection with the embodiments disclosed herein may be implemented as electronic hardware, computer software, or combinations of both. Some of the embodiments and implementations are described above in terms of functional and/or logical block components (or modules) and various processing steps.

However, it should be appreciated that such block components (or modules) may be realized by any number of hardware, software, and/or firmware components configured to perform the specified functions. To clearly illustrate the interchangeability of hardware, various illustrative components, blocks, modules, and steps have been described above generally in terms of their functionality. Whether such functionality is implemented as hardware or software depends upon the application and design constraints imposed on the overall system.

Skilled artisans may implement the described functionality in varying ways for each application, but such implementation decisions should not be interpreted as causing a departure from the scope of the present invention. In addition, those skilled in the art will appreciate that the embodiments described herein are merely exemplary implementations.

In this document, relational terms such as first and second, and the like may be used solely to distinguish one entity or action from another entity or action without necessarily requiring or implying any actual such relationship or order between such entities or actions. Numerical ordinals such as "first," "second," "third," etc. simply denote different singles of a plurality and do not imply any order or sequence unless specifically defined by the claim language. The sequence of the text in any of the claims does not imply that process steps must be performed in a temporal or logical order according to such sequence unless it is specifically defined by the language of the claim. When "or" is used herein, it is the logical or mathematical or, also called the "inclusive or." Accordingly, A or B is true for the three cases: A is true, B is true, and A and B are true. In some cases, the exclusive "or" is constructed with "and;" for example, "one from the set A and B" is true for the two cases: A is true, and B is true.

Furthermore, depending on the context, words such as "connect" or "coupled to" used in describing a relationship between different elements do not imply that a direct physical connection must be made between these elements. For example, two elements may be connected to each other physically, electronically, logically, or in any other manner, through one or more additional elements.

While at least one exemplary embodiment has been presented in the foregoing detailed description of the invention, it should be appreciated that a vast number of variations exist. It should also be appreciated that the exemplary embodiment or exemplary embodiments are only examples and are not intended to limit the scope, applicability, or configuration of the invention in any way. Rather, the foregoing detailed description will provide those skilled in the art with a convenient road map for implementing an exemplary embodiment of the invention. It is understood that various changes may be made in the function and arrangement of elements described in an exemplary embodiment without departing from the scope of the invention as set forth in the appended claims.

## Claims

1. An apparatus comprising a leaflet resection catheter for a Transcatheter Aortic Valve Replacement, TAVR, procedure, the apparatus comprising:
a catheter configured at a distal end with a guidewire to deploy the catheter within a vessel lumen to a situs of an aortic valve for leaflet resection;
an accessory tool (20) configured with a set of grasping elements (22, 24) attached at the catheter's distal end to enable the accessory tool to travel down the vessel lumen to the situs of the aortic valve to exert a pulling action to draw an aortic valve leaflet in a direction towards the catheter's distal end to draw a portion of the aortic valve leaflet into a protective sleeve of the catheter at the catheter's distal end; and
a ring of fibers surrounding the catheter's distal end , said ring of fibers being configured to transmit laser energy to resect tissue that comprises a portion of the aortic valve leaflet drawn into the protective sleeve at the catheter's distal end,
wherein the catheter comprises a guidewire lumen (10) for receiving the guidewire and an accessory tool lumen for receiving the accessory tool (20) configured to travel down the accessory tool lumen.

2. The apparatus of claim 1, wherein the ring of fibers is configured to transmit laser energy to ablate tissue whilst the aortic valve leaflet is held by the set of grasping elements within the protective sleeve to prevent tissue damage outside the protective sleeve.

3. The apparatus of claim 1, wherein the accessory tool is configured to, in response to pulling action while grasping an aortic valve leaflet tissue between the set of grasping elements, enable a reactive action of a simultaneous pulling of the leaflet in a direction into the protective sleeve whilst the distal end of the catheter is motioned in an opposite direction towards the aortic valve leaflet that is drawn into the protective sleeve at the catheter's distal end.

4. The apparatus of claim 3, wherein the accessory tool is configured to provide a vacuum suction for grasping the aortic valve leaflet during ablation of tissue of the aortic valve leaflet.

5. The apparatus of claim 3, wherein the ring of fibers (32) is configured about a section or an entirety of the catheter's distal end to ablate tissue of the aortic valve leaflet.

6. The apparatus of claim 5, wherein the ring of fibers is configured in a plurality of arrangements comprising at least a semicircle, a one-third circle, or another fractional circle of a circumference of the catheter's distal end to ablate tissue of the aortic valve leaflet.

7. The apparatus of claim 3, wherein the ring of fibers is configured to reshape a situs comprising a valvular structure to fit a replacement valve at the situs.

8. The apparatus of claim 3, further comprising a laser and a power source connected at a proximal end of the catheter and configured to energize the laser for ablating the tissue of the aortic valve leaflet.

9. The apparatus of claim 1, wherein the set of grasping elements of the accessory tool at the catheter's distal end is configured to grasp an aortic valve leaflet between each grasping element of the set of grasping elements.

10. The apparatus of claim 1, wherein the ring of fibers is configured to transmit laser energy to ablate a native and
a bioprosthetic valve leaflet.

11. The apparatus of claim 1, further comprising:
a hemostatic valve configured on a proximal end of the catheter to enable the accessory tool to pass through the vessel lumen whilst sealing off arterial pressures.

12. The apparatus of claim 1, further comprising: an introducer of at least a range of 14f to 18f, wherein the catheter is configured to pass through the introducer.

13. The apparatus of claim 1, wherein each fiber (32) of the ring of fibers is configured to transmit laser energy configured to perform tissue resection of tissue, which comprises a portion of the aortic valve leaflet that has been drawn within the protective sleeve and held between the set of grasping elements.

14. The apparatus of claim 1,
wherein the accessory tool (20) is configured to grasp the leaflet between the set of grasping elements (22,24) at the catheter's distal end and to exert pulling action to draw the leaflet towards the catheter's distal end; wherein, in response to the pulling action of the leaflet, the apparatus is configured to draw a portion of the leaflet drawn into the protective sleeve; and wherein each fiber of the ring is configured to transmit laser energy to perform tissue resection of the leaflet portion contained within the protective sleeve.

15. The apparatus of claim 14, wherein the ring of
fibers is configured to ablate via transmissions of the laser to tissue whilst the leaflet is held by the set of grasping elements within the protective sleeve, thereby preventing tissue damage outside the protective sleeve.

16. The apparatus of claim 15, the catheter being configured, in response to the pulling action of the accessory tool, while grasping leaflet tissue between the set of grasping elements, to exhibit a pull-push action to simultaneously pull the leaflet in a direction towards the protective sleeve whilst moving the distal end of the catheter in an opposite direction towards the leaflet and drawing the protective sleeve over the leaflet at the catheter's distal end.

17. The apparatus of claim 16, wherein the ring of fibers is configured about a circumference of a catheter's distal end in entirety to ablate tissue of the leaflet.

18. The apparatus of claim 16, wherein the ring of fibers is configured about a part of the catheter's distal end to ablate tissue of the leaflet.

19. The apparatus of claim 18, wherein the ring of fibers comprises at least a semicircle, a one-third circle, or another fractional circle of the circumference of the catheter's distal end to ablate tissue of the leaflet.

## Patentansprüche

1. Einrichtung, die einen Segelresektionskatheter für einen Transkatheter-Aortenklappenersatz-, TAVR-Vorgang umfasst, wobei die Einrichtung umfasst:
einen Katheter, der an einem distalen Ende mit einem Führungsdraht konfiguriert ist, um den Katheter innerhalb eines Gefäßlumens an einer Stelle einer Aortenklappe zur Segelresektion einzusetzen;
ein Zubehörwerkzeug (20), das mit einem Satz an Greifelementen (22, 24) konfiguriert ist, die am distalen Ende des Katheters angebracht sind, um es dem Zubehörwerkzeug zu ermöglichen, sich entlang des Gefäßlumens zur Stelle der Aortenklappe zu bewegen, um eine Zugaktion auszuüben, um ein Aortenklappensegel in eine Richtung zum distalen Ende des Katheters hin zu ziehen, um einen Abschnitt des Aortenklappensegels in eine Schutzhülle des Katheters am distalen Ende des Katheters zu ziehen; und
einen Faserring, der das distale Ende des Katheters umgibt, wobei der Faserring konfiguriert ist, um Laserenergie zu übertragen, um Gewebe, das einen Abschnitt des in die Schutzhülle am distalen Ende des Katheters gezogenen Aortenklappensegels umfasst, zu resezieren,
wobei der Katheter ein Führungsdrahtlumen (10) zum Aufnehmen des Führungsdrahts und ein Zubehörwerkzeuglumen zum Aufnehmen des Zubehörwerkzeugs (20), das konfiguriert ist, um sich durch das Zubehörwerkzeuglumen zu bewegen, umfasst.

2. Einrichtung nach Anspruch 1, wobei der Faserring konfiguriert ist, um Laserenergie zu übertragen, um Gewebe abzutragen, während das Aortenklappensegel durch den Satz an Greifelementen innerhalb der Schutzhülle gehalten wird, um Gewebeschädigung außerhalb der Schutzhülle zu verhindern.

3. Einrichtung nach Anspruch 1, wobei das Zubehörwerkzeug konfiguriert ist, um als Reaktion auf eine Zugaktion, während es ein Aortenklappensegelgewebe zwischen dem Satz an Greifelementen greift, eine reaktive Aktion eines gleichzeitigen Ziehens des Segels in eine Richtung in die Schutzhülle zu ermöglichen, während das distale Ende des Katheters in eine entgegengesetzte Richtung zum Aortenklappensegel hin bewegt wird, das am distalen Ende des Katheters in die Schutzhülle gezogen wird.

4. Einrichtung nach Anspruch 3, wobei das Zubehörwerkzeug konfiguriert ist, um einen Vakuumsog zum Greifen des Aortenklappensegels während eines Abtragens von Gewebe des Aortenklappensegels bereitzustellen.

5. Einrichtung nach Anspruch 3, wobei der Faserring (32) um ein Teilstück oder eine Gänze des distalen Endes des Katheters konfiguriert ist, um Gewebe des Aortenklappensegels abzutragen.

6. Einrichtung nach Anspruch 5, wobei der Faserring in einer Vielzahl von Anordnungen konfiguriert ist, die mindestens einen Halbkreis, einen Drittelkreis oder einen anderen Teilkreis eines Umfangs des distalen Endes des Katheters umfassen, um Gewebe des Aortenklappensegels abzutragen.

7. Einrichtung nach Anspruch 3, wobei der Faserring konfiguriert ist, um eine Stelle, die eine Klappenstruktur umfasst, umzuformen, sodass sie zu einer Ersatzklappe an der Stelle passt.

8. Einrichtung nach Anspruch 3, die weiter einen Laser und eine Leistungsquelle umfasst, die an einem proximalen Ende des Katheters angeschlossen und konfiguriert ist, um den Laser zum Abtragen des Gewebes des Aortenklappensegels mit Energie zu versorgen.

9. Einrichtung nach Anspruch 1, wobei
der Satz an Greifelementen des Zubehörwerkzeugs am distalen Ende des Katheters konfiguriert ist, um zwischen jedem Greifelement des Satzes an Greifelementen ein Aortenklappensegel zu greifen.

10. Einrichtung nach Anspruch 1, wobei der Faserring konfiguriert ist, um Laserenergie zu übertragen, um ein natives und ein bioprothetisches Klappensegel abzutragen.

11. Einrichtung nach Anspruch 1, weiter umfassend:
ein hämostatisches Ventil, das an einem proximalen Ende des Katheters konfiguriert ist, um dem Zubehörwerkzeug zu ermöglichen, durch das Gefäßlumen hindurchzutreten, während es arterielle Drücke abdichtet.

12. Einrichtung nach Anspruch 1, weiter umfassend:
eine Einführvorrichtung mindestens eines Bereichs von 14f bis 18f, wobei der Katheter konfiguriert ist, um durch die Einführvorrichtung hindurchzutreten.

13. Einrichtung nach Anspruch 1, wobei jede Faser (32) des Faserrings konfiguriert ist, um Laserenergie zu übertragen, die konfiguriert ist, um Geweberesektion von Gewebe durchzuführen, das einen Abschnitt des Aortenklappensegels umfasst, das in die Schutzhülle gezogen wurde und zwischen dem Satz an Greifelementen gehalten wird.

14. Einrichtung nach Anspruch 1,
wobei das Zubehörwerkzeug (20) konfiguriert ist, um das Segel zwischen dem Satz an Greifelementen (22, 24) am distalen Ende des Katheters zu greifen und Zugaktion auszuüben, um das Segel in Richtung des distalen Endes des Katheters zu ziehen; wobei die Einrichtung konfiguriert ist, um als Reaktion auf die Zugaktion des Segels einen Abschnitt des in die Schutzhülle gezogenen Segels zu ziehen; und wobei jede Faser des Rings konfiguriert ist, um Laserenergie zu übertragen, um Geweberesektion des innerhalb der Schutzhülle enthaltenen Segelabschnitts durchzuführen.

15. Einrichtung nach Anspruch 14, wobei der Faserring konfiguriert ist, um durch Übertragungen des Lasers auf Gewebe abzutragen, während das Segel durch den Satz an Greifelementen innerhalb der Schutzhülle gehalten wird, wodurch Gewebeschädigung außerhalb der Schutzhülle verhindert wird.

16. Einrichtung nach Anspruch 15, wobei der Katheter konfiguriert ist, um als Reaktion auf die Zugaktion des Zubehörwerkzeugs, während es Segelgewebe zwischen dem Satz an Greifelementen greift, eine Zug-/Schubaktion auszuüben, um gleichzeitig das Segel in eine Richtung zur Schutzhülle hin zu ziehen, während er das distale Ende des Katheters in eine entgegengesetzte Richtung zum Segel hin bewegt und die Schutzhülle über das Segel am distalen Ende des Katheters zieht.

17. Einrichtung nach Anspruch 16, wobei der Faserring um einen Umfang des distalen Endes eines Katheters in Gänze konfiguriert ist, um Gewebe des Segels abzutragen.

18. Einrichtung nach Anspruch 16, wobei der Faserring um einen Teil des distalen Endes des Katheters konfiguriert ist, um Gewebe des Segels abzutragen.

19. Einrichtung nach Anspruch 18, wobei der Faserring mindestens einen Halbkreis, einen Drittelkreis oder einen anderen Teilkreis des Umfangs des distalen Endes des Katheters umfasst, um Gewebe des Segels abzutragen.

## Revendications

1. Appareil comprenant un cathéter de résection de feuillet pour une intervention de remplacement transcathéter de valvule aortique, TAVR, l'appareil comprenant :
un cathéter configuré au niveau d'une extrémité distale avec un fil-guide pour déployer le cathéter à l'intérieur d'une lumière de vaisseau jusqu'à un site d'une valvule aortique en vue d'une résection de feuillet ;
un outil accessoire (20) configuré avec un ensemble d'éléments de saisie (22, 24) fixés au niveau de l'extrémité distale du cathéter pour permettre à l'outil accessoire de descendre le long de la lumière de vaisseau jusqu'au site de la valvule aortique afin d'exercer une action de traction pour tirer un feuillet de valvule aortique dans une direction vers l'extrémité distale du cathéter pour tirer une partie du feuillet de valvule aortique dans un manchon de protection du cathéter au niveau de l'extrémité distale du cathéter ; et
un anneau de fibres entourant l'extrémité distale du cathéter, ledit anneau de fibres étant configuré pour transmettre de l'énergie laser pour réséquer un tissu qui comprend une partie du feuillet de valvule aortique tirée dans le manchon de protection au niveau de l'extrémité distale du cathéter,
dans lequel le cathéter comprend une lumière de fil-guide (10) pour recevoir le fil-guide et une lumière d'outil accessoire pour recevoir l'outil accessoire (20) configuré pour descendre le long de la lumière d'outil accessoire.

2. Appareil selon la revendication 1, dans lequel l'anneau de fibres est configuré pour transmettre de l'énergie laser pour effectuer l'ablation du tissu en même temps que le feuillet de valvule aortique est maintenu par l'ensemble d'éléments de saisie à l'intérieur du manchon de protection pour empêcher tout dommage tissulaire à l'extérieur du manchon de protection.

3. Appareil selon la revendication 1, dans lequel l'outil accessoire est configuré pour, en réponse à une action de traction lors de la saisie d'un tissu de feuillet de valvule aortique entre l'ensemble d'éléments de saisie, permettre une action réactive d'une traction simultanée du feuillet dans une direction dans le manchon de protection en même temps que l'extrémité distale du cathéter est déplacée dans une direction opposée vers le feuillet de valvule aortique qui est tiré dans le manchon de protection au niveau de l'extrémité distale du cathéter.

4. Appareil selon la revendication 3, dans lequel l'outil accessoire est configuré pour fournir une aspiration sous vide pour saisir le feuillet de valvule aortique pendant l'ablation de tissu du feuillet de valvule aortique.

5. Appareil selon la revendication 3, dans lequel l'anneau de fibres (32) est configuré autour d'une section ou d'une totalité de l'extrémité distale du cathéter pour effectuer l'ablation du tissu du feuillet de valvule aortique.

6. Appareil selon la revendication 5, dans lequel l'anneau de fibres est configuré dans une pluralité d'agencements comprenant au moins un demi-cercle, un tiers de cercle ou une autre fraction de cercle d'une circonférence de l'extrémité distale du cathéter pour effectuer l'ablation du tissu du feuillet de valvule aortique.

7. Appareil selon la revendication 3, dans lequel l'anneau de fibres est configuré pour redéfinir un site comprenant une structure valvulaire pour s'adapter à une valvule de remplacement au niveau du site.

8. Appareil selon la revendication 3, comprenant en outre un laser et une source d'énergie connectée au niveau d'une extrémité proximale du cathéter et configurée pour activer le laser afin d'effectuer l'ablation du tissu du feuillet de valvule aortique.

9. Appareil selon la revendication 1, dans lequel
l'ensemble d'éléments de saisie de l'outil accessoire au niveau de l'extrémité distale du cathéter est configuré pour saisir un feuillet de valvule aortique entre chaque élément de saisie de l'ensemble d'éléments de saisie.

10. Appareil selon la revendication 1, dans lequel l'anneau de fibres est configuré pour transmettre de l'énergie laser pour effectuer l'ablation d'un feuillet de valvule natif et bioprothétique.

11. Appareil selon la revendication 1, comprenant en outre :
une valve hémostatique configurée sur une extrémité proximale du cathéter pour permettre à l'outil accessoire de traverser la lumière de vaisseau tout en assurant l'étanchéité vis-à-vis des pressions artérielles.

12. Appareil selon la revendication 1, comprenant en outre :
un introducteur d'au moins une plage de 14f à 18f, dans lequel le cathéter est configuré pour passer à travers l'introducteur.

13. Appareil selon la revendication 1, dans lequel chaque fibre (32) de l'anneau de fibres est configurée pour transmettre de l'énergie laser configurée pour effectuer une résection tissulaire d'un tissu, qui comprend une partie du feuillet de valvule aortique qui a été tirée à l'intérieur du manchon de protection et maintenue entre l'ensemble d'éléments de saisie.

14. Appareil selon la revendication 1,
dans lequel l'outil accessoire (20) est configuré pour saisir le feuillet entre l'ensemble d'éléments de saisie (22, 24) au niveau de l'extrémité distale du cathéter et pour exercer une action de traction pour tirer le feuillet vers l'extrémité distale du cathéter ; dans lequel, en réponse à l'action de traction du feuillet, l'appareil est configuré pour tirer une partie du feuillet tiré dans le manchon de protection ; et dans lequel chaque fibre de l'anneau est configurée pour transmettre de l'énergie laser pour effectuer une résection tissulaire de la partie de feuillet contenue dans le manchon de protection.

15. Appareil selon la revendication 14, dans lequel l'anneau de fibres est configuré pour effectuer une ablation via des transmissions du laser au tissu en même temps que le feuillet est maintenu par l'ensemble d'éléments de saisie à l'intérieur du manchon de protection, empêchant ainsi tout dommage tissulaire à l'extérieur du manchon de protection.

16. Appareil selon la revendication 15, le cathéter étant configuré, en réponse à l'action de traction de l'outil accessoire, tout en saisissant le tissu de feuillet entre l'ensemble d'éléments de saisie, pour présenter une action de traction-poussée pour tirer simultanément le feuillet dans une direction vers le manchon de protection tout en déplaçant l'extrémité distale du cathéter dans une direction opposée vers le feuillet et en tirant le manchon de protection par-dessus le feuillet au niveau de l'extrémité distale du cathéter.

17. Appareil selon la revendication 16, dans lequel l'anneau de fibres est configuré autour d'une circonférence de l'extrémité distale d'un cathéter dans son intégralité pour effectuer l'ablation du tissu du feuillet.

18. Appareil selon la revendication 16, dans lequel l'anneau de fibres est configuré autour d'une partie de l'extrémité distale du cathéter pour effectuer l'ablation du tissu du feuillet.

19. Appareil selon la revendication 18, dans lequel l'anneau de fibres comprend au moins un demi-cercle, un tiers de cercle ou une autre fraction de cercle de la circonférence de l'extrémité distale du cathéter pour effectuer l'ablation du tissu du feuillet.
